# EUROPEAN PATENT SPECIFICATION

(11) **EP 0 991 756 B1**
(45) Date of publication and mention of the grant of the patent: **04.04.2007**
(21) Application number: 97948755.0
(22) Date of filing: 22.08.1997
(51) Int. Cl.: C12N 15/12, C07K 14/82, C07K 14/47, C12N 15/11, A61K 38/17, C07K 16/32, C12Q 1/68, G01N 33/68

(54) **AGENTS FOR THE PRE-SYMPTOMATIC DETECTION, PREVENTION AND TREATMENT OF BREAST CANCER IN HUMANS**
Agentien zur präsymptomatischen Detektion, Prävention und Behandlung von Brustkrebs in Menschen
AGENTS PERMETTANT LA DETECTION, LA PREVENTION, ET LE TRAITEMENT, AVANT APPARITION DES SYMPTOMES, DU CANCER DU SEIN CHEZ LES HUMAINS

(30) Priority: 22.08.1996 CA 2183900
(43) Date of publication of application: 12.04.2000
(73) Proprietor: BERGMANN, Johanna, E., D-22587 Hamburg (DE); Preddie, Rick, E., 22587 Hamburg (DE)
(72) Inventor: BERGMANN, Johanna, E., D-22587 Hamburg (DE); Preddie, Rick, E., 22587 Hamburg (DE)
(86) International application number: PCT/EP1997/004600
(87) International publication number: WO 1998/007851

(56) References cited:
- WO-A-94/21791
- HARLOW E ET AL: "MOLECULAR CLONING AND IN VITRO EXPRESSION OF A CDNA CLONE FOR HUMANCELLULAR TUMOR ANTIGEN P53" MOLECULAR AND CELLULAR BIOLOGY, vol. 5, no. 7, July 1985, pages 1601-1610, XP000619201
- MIKI Y ET AL: "A STRONG CANDIDATE FOR THE BREAST AND OVARIAN CANCER SUSCEPTIBILITY GENE BRCA1" SCIENCE, vol. 266, no. 5182, 7 October 1994, pages 66-71, XP002024010 cited in the application
- MERINO, E. ET AL.: "Antisense overlapping open reading frames in genes from bacteria to humans" NUCLEIC ACIDS RESEARCH., vol. 22, 1994, OXFORD GB, pages 1903-1908, XP002066206

## Description

### Field of the Invention:

The invention relates to diagnostic reagents with therapeutic potential for the detection, prevention and treatment of breast and ovarian cancer, in humans. More specifically, the invention relates to the gene. BREX, that is implicated in causing breast cancer, to its expressed protein product and the use of this protein as diagnostic probe for pre-symptomatic detection of breast and associated cancers. The invention additionally relates to nucleic acid molecules that influence the expression of this gene. The invention also relates to first and second medical uses of these reagents.

### Cross reference to Related Applications:

This application is related to PCT/EP94/00651, WIPO publication Number WO 94/21791.

### Background of the Invention:

### I. Breast Cancer

Breast cancer affects one in ten women and one in four thousand men. About five percent of these cases in women (.025% in men) are associated with germ line heritable mutations. The rest, believed to be caused by somatic mutations of mostly unidentified genes, are termed "sporadic". As an inherited trait breast cancer is one of the most common genetic diseases in the industrial world; in fact, one out of 100 women alive today will develop breast cancer due to inheritance unless a cure for the disease is found. About 40% of cases are diagnosed before the patient has attained the age of 30.

At the moment there is neither a cure for breast cancer nor a non-invasive method for early detection of the disease. Breast cancer is presently treated using surgery, endocrine therapy and chemotherapy (Salmon, S.E., Semin. Oncol., 17:50-52 (1990); Hortobagyi, G.N., Breast Cancer Res. Treat 21:3-13 (1992)). Endocrine therapy results in complete or partial remissions in only 30% of patients (Jiang, S.-Y. et al., J. Natl. Canc. Inst. 84:580-591 (1992); Muss, H.B., Breast Cancer Res. Treat 21:15-26 (1992)). Chemotherapy, despite the development of new antineoplastic agents, has had only limited success in treating breast cancer (Hortobagyi, G.N., Breast Cancer Res. Treat 21:3-13 (1992)). Thus, surgery is the only presently proven treatment for breast cancer; its use has long been controversial (Albert, S. et al., Cancer 41:2399-2408 (1978). Surgery is often combined with endocrine therapy or chemotherapy regimes.

Biological agents, such as interferon and interleukin, have been found to be capable of producing definite anti-tumour responses. Unfortunately, such advances have not yet led to improved regimens for managing breast cancer (Hortobagyi, G.N., Breast Cancer Res. Treat 21:3-13 (1992)). Monoclonal antibodies have also been employed to help the natural immune system. However, the antibodies available to date have not had the specificity or sensitivity to successfully target tumour cells.

The above approaches were based mainly on two overlapping hypotheses about the origin of breast cancer. One is that breast cancer originates in the breast and metastasizes serially through lymph nodes before spreading to the rest of the body. This was the impetus for use of radical mastectomy combined with very early detection. The second hypothesis proposes that the disease invades the blood stream very early and spreads slowly (8 - 10 years) before it is detected in the breast by mammography or other specialized examination. This spurred the use of chemical adjuvant chemotherapy which did not improve the survival rate. Although these thoughts still pervade clinical approaches to breast cancer management, it is becoming increasingly obvious that killing tumour cells is not the answer but in fact the environment of the cell must be changed. This has to be done, essentially, by unravelling and manipulating the molecular events which control the expression of, until now, unknown genes which have disruptive effects on cell growth and development. One environmental mechanism through which cancer arises is cell exposure to a 'carcinogenic' chemical or to radiation. Such an exposure may damage the DNA leading to either an impairment of expression of a specific gene or the production of a mutant gene, or the expression of a gene which is normally repressed throughout life. The cell may then proceed to proliferate uncontrollably and alternately result in tumour formation.

Two classes of critical genes have been found experimentally to be influenced by the above mentioned conditions. One class of such genes has been referred to as "oncogenes". Oncogenes are genes which are naturally in an "inactivated" state, but which, through the effect of the DNA damage are converted to an "activated" state capable of inducing tumorigenesis (i.e. tumour formation). Oncogenes have been identified in 15-20% of human tumours. The products of oncogenes ("oncoproteins") can be divided into two broad classes according to their location in the cell.

Oncogene products which act in the cytoplasm of cells have readily identifiable biochemical or biological activities (Green, M.R., Cell 56:-3 (1989)). Those that act in the nucleus of a cell have been more difficult to characterize. Some nuclear oncoproteins (such as E1A and myc) have transcriptional regulatory activity, and are believed to mediate their activities by the transcriptional activation of cellular genes (Kingston, R.E., Cell 41;3-5 (1985)). Other nuclear oncoproteins appear to have a complex array of activities (such as DNA binding activity, ability to initiate viral DNA synthesis, ATPase activity, helicase activity, and transcriptional regulatory activity) (Green, M.R., Cell 56:1-3 (1989)).

The second class of genes are the anti-oncogenes, or "tumour-suppressing genes". In their natural state these genes act to suppress factors which cause cell proliferation. Damage to such genes leads to a loss of this suppression, and thereby sometimes results in tumorigenesis. Thus, the deregulation of cell growth, which is a major characteristics of tumour cell proliferation may be mediated by either the activation of oncogenes or the inactivation of tumour-suppressing genes (Weinberg, R.A., Scientific Amer., Sept. 1988, pp 44-51).

Oncogenes and tumour-suppressing genes have a basic distinguishing feature. The oncogenes identified thus far have arisen only in somatic cells, and thus have been incapable of transmitting their effects to the germ line of the host animal. In contrast, mutations in tumour-suppressing genes are identified in germ cell lines and are thus transmissible to the offspring.

Mutations in three tumour-suppressing genes and one putative tumour-suppressing gene have been shown to be involved in inherited breast cancer. To date, human familial breast cancer has been associated with point mutations and deletions in four genes located on three different chromosomes. Three of the four genes, BRCA1 on chromosome 17q21-23 (Miki, Y et al., Science, 265:66-71 1994), TP53 on chromosome 17p13 (Malkin D., et al., Science 250. 1233-1237 1990) and "BRCA2" on chromosome 13q12-13 (Wooster A. et al., Science 265:2088-2090 1994; Nature 579:789-792 (1995)), are involved in female breast and ovarian cancer. The fourth, the androgen receptor on chromosome Xq11-12 (Wooster A., et al., Nature Genet. 2:132-134 1992), and BRCA2 are involved in male breast cancer. The KFLT gene lies in a region close to BRCA2 on chromosome 13 and is a likely candidate for involvement in some aspects of BC. Germ line mutations in the retinoblastoma gene (RB gene) have not been implicated, so far, in breast or ovarian cancer; however, somatic mutation in the RB gene are associated with sporadic breast cancers and somatic mutations in the TP53 gene are associated with sporadic breast and ovarian cancers (T'Ang.J.M., et. al., Science 242:263-265 (1988)). The exclusion of BRCA1 from the nucleus (or the complete absence) has been shown to be associated specifically with all forms of breast cancer (Chen Y. et al., Science 2.70:789-791 (1995)). In spite of the fact that about 56 germ-line mutations have been discovered in the BRCA1 gene (Shattuck-Eidens D. et al., JAMA 273:535-541 (1995)) and about a dozen in BRCA2 (Wooster, R. et al., Nature 579:789-792 (1995)) somatic mutation in these genes do not appear to be associated with breast and/or ovarian cancer (Futreal A., et al., Science, 266: 120-122 (1994).

The odds of an individual developing breast cancer cannot be predicted from genetic analysis, because the absence of a germ cell mutation is no guarantee against becoming a victim of the disease. A woman with normal BRCA1, TP53 and (from what is known so far) BRCA2 genes, has the same risk as a woman with a hereditary mutation in one of these genes, of getting the disease even if in the latter case it occurs a little later in life. In fact there is a high probability that a person with a predisposing hereditary mutation can develop the disease from a factor (presently unknown) unrelated to the mutation.

As mentioned above only about 5% of all breast cancer victims have mutations in, at least, one of the predisposing genes. The other 95% of breast cancers are considered to be of sporadic origin. The high ratio of sporadic to inherited breast cancer (19 to 1) and the fact that there are no clinical or pathological differences in familial versus sporadic breast and ovarian cancer other than the age of onset (Lynch, H.T., et al. Gynecol. Oncol. 36, 48-53 (1990), is a clear indication that mutations in the breast cancer related genes are not direct causes of the pathophysiological symptoms of the disease. Instead, common biochemical factors must be affected by mutations in all predisposing genes and by other "sporadic causes" of the disease. BRCA1 appears to be a common factor in all forms of breast cancer, but neither the disruptions or mutations in BRCA1 is an originating factor. The original factor must act at some point before the tumour suppressing activity of BRCA1/BRCA2 is disrupted. The latter activity may be related to transcriptional activation functions of these two genes (Milner, J et. al. Nature 386,772-773 (1997)).

### SUMMARY OF THE INVENTION

The object of the present invention was to discover naturally occurring molecules which are expressed in a disease specific manner that were useful as specific, universal, diagnostic markers for breast and ovarian cancer (breast cancer) and which were also potential targets for therapeutics to treat breast cancer.

According by the present patent described in approach to discovering disease specific genes which we have developed. The application of this procedure led to the discovery of a number of pathogenic proteins (L-oncoproteins) which are expressed by L-oncogenes. These L-oncogene were located within the chromosomal loci occupied by the tumour suppressor genes described above. These molecules are expressed in humans with the disease but are not expressed in humans not afflicted with breast cancer. In contrast to oncogenes which must be activated by mutation, L-oncogenes are preactivated oncogenes, i.e., the are expressed in the activated form in response to a number of environmental signals. The invention, which is defined by the appended claims, concerns one of these genes, namely BREX, whose sequence is given in SEQ ID NO: 28 and 32, whereas its corresponding protein has the sequence given in SEQ ID NO : 29. agents its use as marker for early diagnosis and target for therapeutic approaches for all forms of human breast cancer. Described are also other novel proteins implicated by unique association with breast cancer as well as analogues and derivatives of these molecules, and nucleic acid molecules encoding such molecules, or influencing their expression.

### BRIEF DESCRIPTION OF THE FIGURES

FIG. 5a (SEQ ID NO:28), shows the cDNA for BREX) which is encoded by sequences homologous to exon 16 of the antisense strand of the BRCA1 gene
FIG. 5b (SEQ ID NO:29), shows the deduced protein (BREX).
FIG. 5d (SEQ ID NO:31) shows the 5' regulatory region of BREX. It contains three TATA boxes correlated to cap sites and a GC box. The sequence is homologous to a region of the anti sense of BRCA1 intron 17.
FIG. 7e Functional homology between the c terminal putative nuclear transit sequence of BC531 and the two putative nuclear transit sequences of BRCA1.
FIG. 7g Domain homology between p53, BREX and FLT4
FIG. 7h Homology between BC532 and BREX
FIG. 8. An identical core promoter sequence "ATAAAA" in at least one promoter element in the regulatory region of each L-oncogenes.
FIG. 9c Organization of BREXL genes

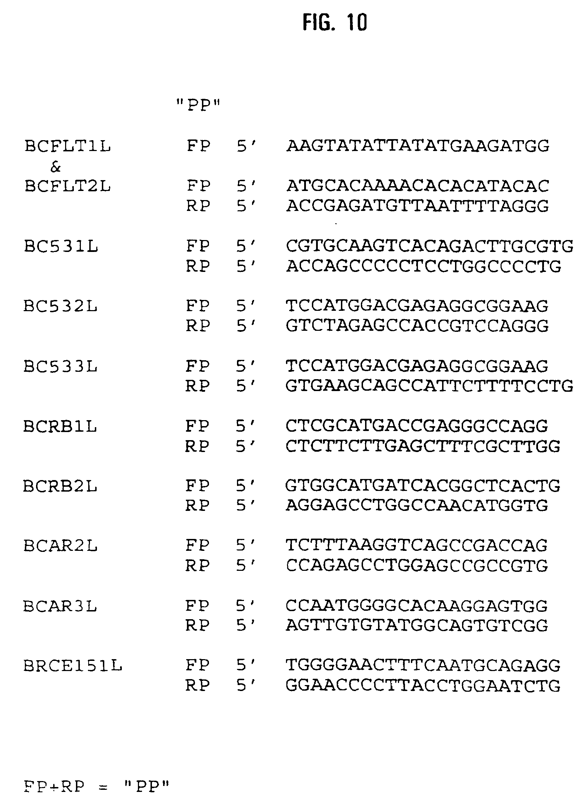

FIG. 10 Sequence of primers used in RT-PCR to detect L-oncogene mRNA in clinical specimens.
FIG. 12a Sequence of epitopes against which mono-specific polyclonal rabbit antibody was raised. The antibodies were used to detect the expression of L-oncoproteins in clinical specimens.
FIG 12b Outline of ELISA antigen trap test.
FIG 12c Outline of ELISA anmtibody trap test.
FIG 12d & e Detection of BC531Lp & BC532 in proteins isolated from breast tumours and normal breast of unrelated individuals.
FIG. 12f & g Detection of BC531 & BC532 in proteins isolated from primary tumours of unrelated women.
FIG. 12h & i Detection of BC531 and BE532 in the nuclear fraction of proteins isolated from advanced breast tumours with lymph node involvement.

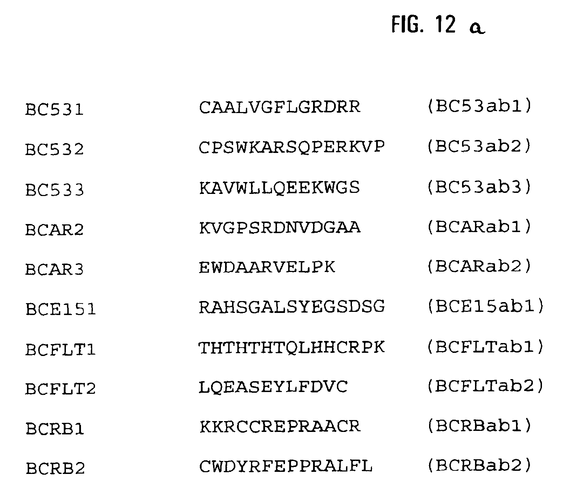

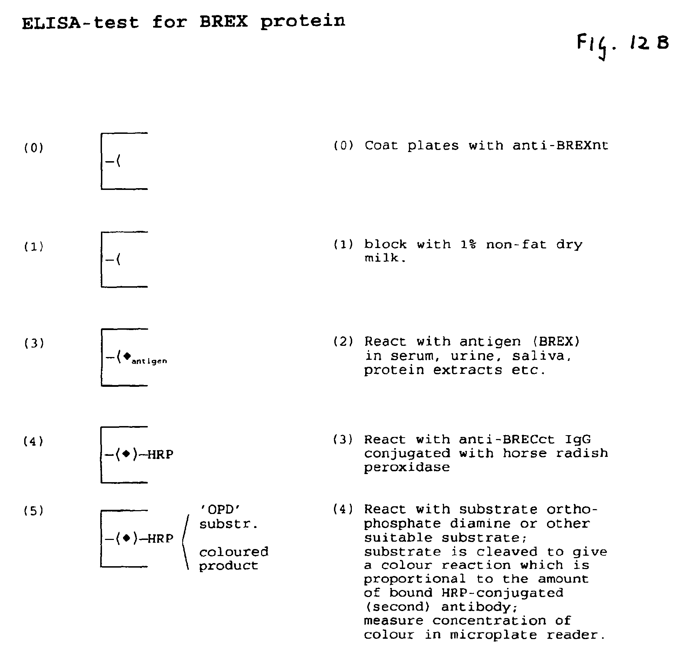

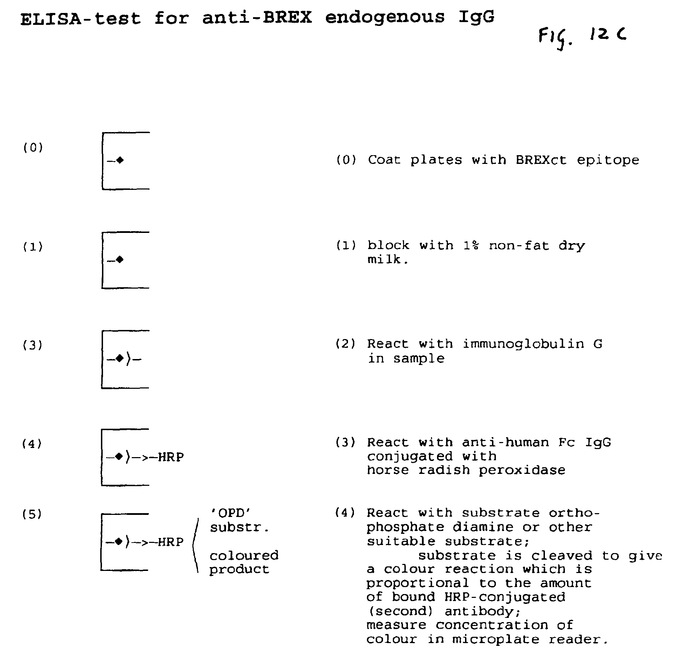

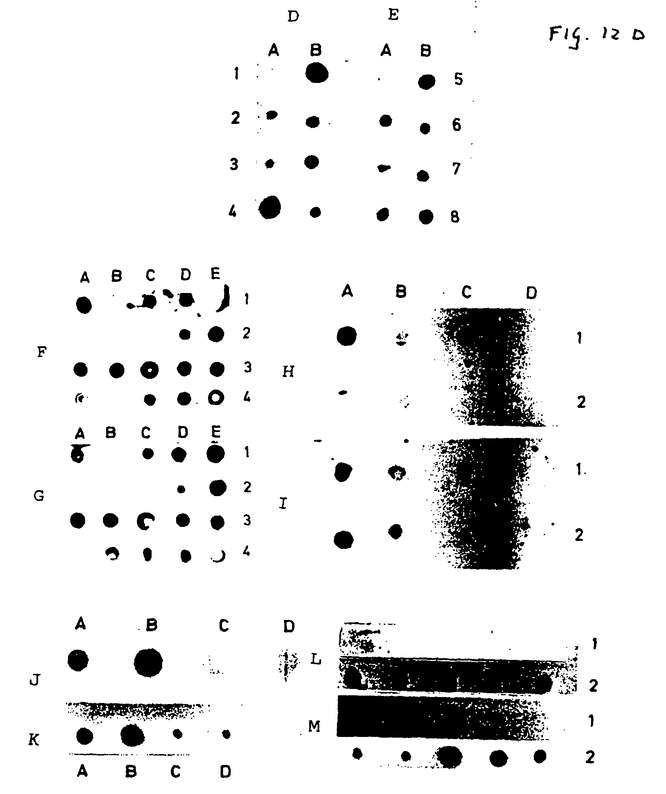

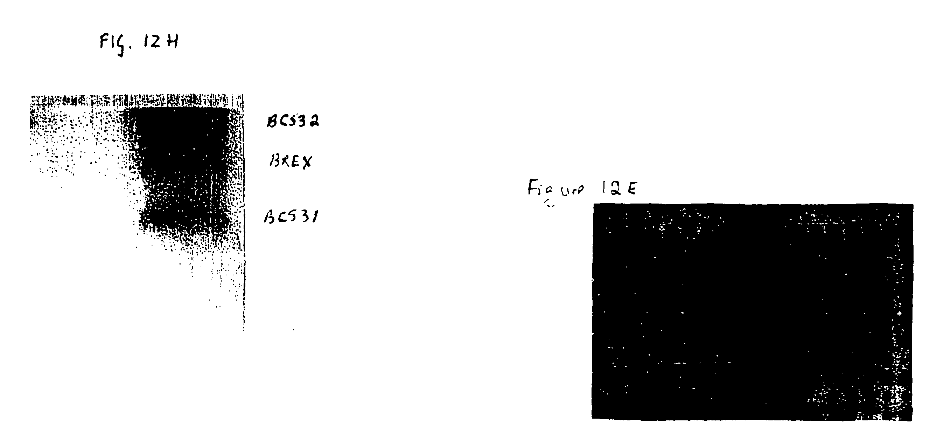

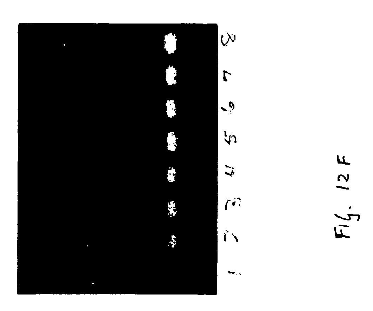

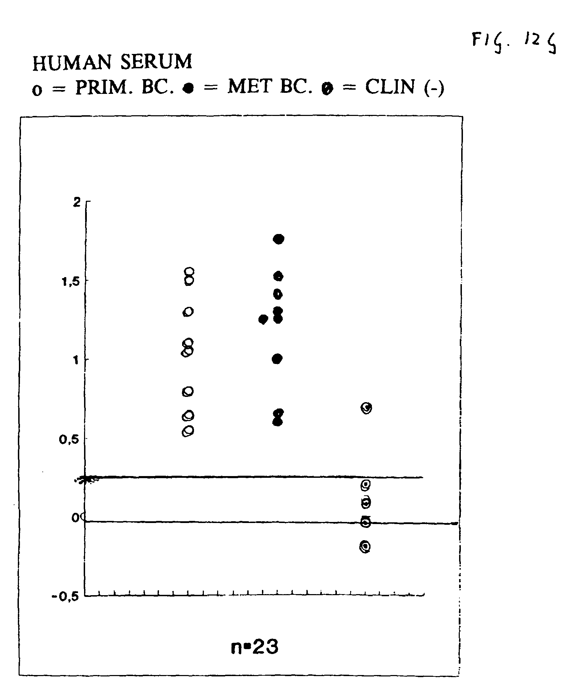

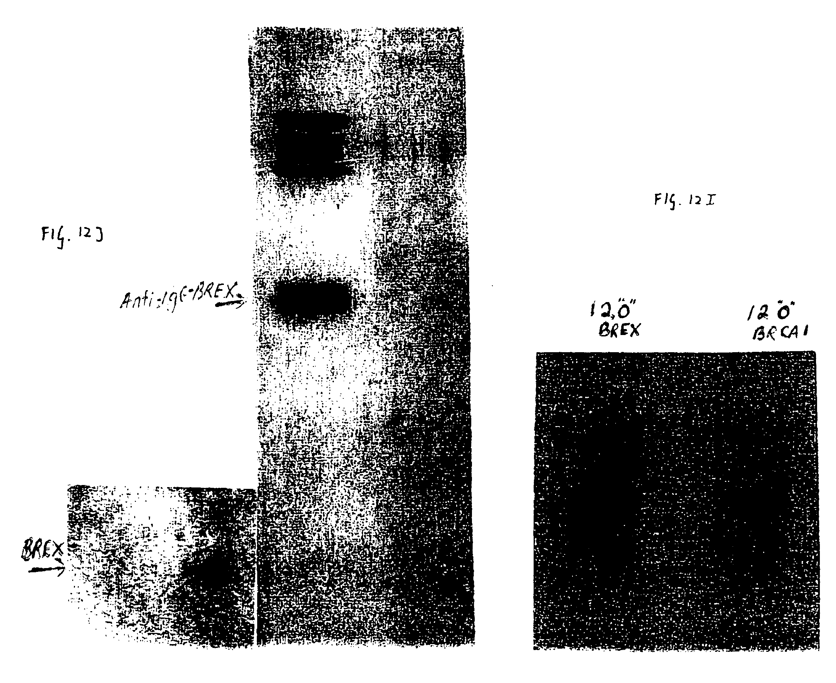

FIG. 12 j detection of BC531 and BC532 in blood obtained from 18 women with tumours less than 1 cm in diameter.
FIG. 12 o Immunodetection of BREX and BRCA1 in breast tumour tissue from the same patient.

### DETAILS OF THE INVENTION.

In detail, the invention provides a nucleic acid molecule, substantially free of natural contaminants, that encodes the protein BREX, whose sequence is SEQ ID NO:28.

The invention also provides the BREX protein, substantially free of natural contaminants, having the sequence of SEQ ID NO:29.

The invention also concerns an antibody, or a fragment of an antibody, which is capable of binding to a molecule having a sequence of SEQ ID NO: 29.

The invention also provides the use of an antisense RNA specific for BREX mRNA or for SEQ ID NO:29 or of an antibody against BREX for the manufacture of a medicament for the prophylactic treatment of breast cancer.

### DESCRIPTION OF THE PREFERRED EMBODIMENTS:

The success of the present invention came mainly from our realization that there must be other genes within the loci occupied bs Tp53 and BRCA1 which were associated with the biochemistry of breast and ovarian cancer, and that at least one of these genes must also have a related component on other genes known to be associated with the disease. Therefore, we used a procedure which we had successfully used to find alternative genes which were putative causative factors of other "genetic diseases", to search for such genes which might segregate with breast cancer within the loci encoding Tp53 gene on chromosome 17. We then used the potential genes detected in this regions as probes to scan sequences on the BRCA1 gene, the RB gene, the AR gene, and sequenced regions in the proposed "BRCA2" locus on chromosome 13, which were available to us, to look for similar sequences.

We call the above mentioned (alternative genes) "piggy-back genes" (pb genes), and we refer to this technology as "disease gene discovery by positional searching" (DGDPS). As a working definition, pb genes are transcribed in any orientation within the chromosomal locus occupied by another characterised functional gene.

### Following is a description of DGDPS procedure.

This procedure has an advantage over gene isolation by cloning from a genomic or cDNA library, because it overcomes three important drawbacks, (1) the possibility that some DNA sequences cannot be cloned by the conventional methods, (2) that some mRNA sequences are of such low abundance that they are not represented in the cDNA library, and (3) the products of some cloned sequences are highly toxic to bacterial or other hosts.

In general, first we identified a gene closely related to a gene already genetically linked to a certain disease, then isolated the mRNA transcribed from the gene from disease tissue or patient's blood, then synthesized cDNA from the isolated mRNA with reverse transcriptase then amplified the novel cDNA with specific primers which flanked the entire coding region of the cDNA, then we identified the cDNA from the size following electrophoresis on agarose gel, and finally isolated the unique cDNA from the agarose gel. This allowed us to select out the desired molecule, if it was expressed, without having to probe several hundred thousand cDNA clones.

The present invention derives from the discovery and cloning of novel gene, a BREXL, using the above approach. Following are examples of positional searching as it was applied in the present invention:
(1) We examined the sequenced regions within the p53 and BRCA1 loci on chromosome 17 and selected potential complete "orfs", i.e. with orfs acceptable translation initiation sequences (see Kozak, M. Nucleic Acid Res. 12:857-872 1984) and translation termination stop codons (TAA, TAG or TGA) in place,
(2) next we used the method of Bucher et al., J. Mol. Biol. 212; 563 - 578 (1990) to identify putative promoter regions associated with the orf within 100-1000 bp 5' upstream of the translation initiation sequence and we identified potential poly-A addition signals (the consensus poly-A addition sequence is "AATAAA") within a region of ~1000 bp 3' downstream from the stop translation codon of the potential orf,
(3) then, orfs fulfilling the above two characteristics were translated into putative protein sequences using the universal code,
(4) then we analyzed the putative protein with our proprietary computer assisted protein finger printing technology and obtained information about the potential biochemical characteristics of the deduced proteins,
(5) next the biochemical characteristics of the deduced proteins were correlated with the known biochemical characteristics of breast cancer,
(6) next RNA encoding proteins with properties correlating with the disease characteristics were selected as potential disease related candidates,
(7) next transcription of the selected sequences was investigated in clinical material. [detection of the presence of transcribed mRNA sequences encoding the protein in a cell can be determined by any means capable of detecting mRNA.
   In this invention reverse transcriptase-PCR (RT-PCR) was done using the Stratagene RAP-PCR RT-PCR kit according to the manufacturer's instructions, with unlabelled primers to detect cDNAs encoding the deduced proteins in RNA isolated from frozen human breast tumours, normal breast and lymphocytes. RNA from frozen tissue was extracted by grinding about 20 mg frozen breast tissue in a tissue homogenizer in the presence of diethylpyrocarbonate (DEPC). Total RNA was isolated using the Stratagene micro RNA isolating Kit and poly (A)+ was isolated using Stratagene Poly(A)+ Quick mRNA isolation kit following the manufacturer's instruction. Isolated RNA was treated with 10 units of RNAse free DNAse at 37oC for 15 minutes. DNAse was inactivated by treating for 2 mins at 100 oC. cDNA synthesis using mRNA as template was carried out with the first strand protocol supplied with the and RT-PCR was done using the cycling conditions recommended by the manufacturer. Forward and reverse PCR primers were prepared to regions flanking the entire protein coding region of the orf of the selected protein (see table 1 for sequence of the primers and for the size of the expected amplified product). The amplified cDNA was electrophoresed on agarose gels and the size was determined by comparison with DNA size markers which were electrophoresed along side. To verify the sequence of the cDNA, the region of agarose containing the desired size cDNA was extracted into H2O, precipitated with ethanol and a portion was cycle sequenced using the primers in "12" and Perkin Elmer ampli-Taq on the Perkin Elmer 376 A DNA sequencer using a non radioactive method described by Liu, C. et al.Nucl. Acid. Res 21:333-334 (1993).
(8) next it was determined that the proteins were actually expressed: in order to do this epitopes were identified within the amino acid sequence of the deduced protein using the method of Hopp, T.P. and Woods, K.R. Proc. Natl Acad Sci. USA 78:3824-3828 (1981) and their sequences compared to sequences in databases; epitopes (see table #2) having no homologue with sequences in public databases were selected. Mono-specific polyclonal rabbit antibodies were prepared against these and purified by immunoaffinity chromatography on Pharmacia LKB, CNBr-activated sepharose 4B according to the recommendation of the manufacturer (see section #11 on Immumology, in Current Protocols in Molecular Biology (Vol 1) Ausubel, F.M. et al (ed) John Wiley & Sons NY. NY. 1991). Enzyme-based immunoassay formats (ELISAs) were done with a colorimetric assay using horse radish peroxidase conjugated IgG as the second antibody and orthophosphate diamine (OPD) as substrate (see "ELISA and other Solid Phase Immunoassays" (Kemeny, D.M. et al. (eds) John Wiley & sons, N.Y, N.Y (1988). Two versions of ELISA were used to determine the presence of the specific breast cancer related proteins in body fluids (blood, serum, saliva) obtained from breast cancer patients and the presence of an endogenous IgG produced by all breast cancer patients against these proteins.
   To detect the expression of the deduced proteins in human material, proteins were isolated from breast tumours and normal tissue by precipitating the homogenized tissue with >80% ammonium sulphate and dialysis against SDSPAGE buffer (the buffer conditions are described in Laemmeli, U.K. Nature 227:680-685 (1970)). Dialysed proteins were boiled in SDSPAGE buffer and either electrophoresed in 17.5% acrylamide gel, following which the proteins were Western blotted onto nylon membrane and treated with the affinity purified antibody or spotted onto positively charged membranes and treated as above. Interaction of the antibody with the protein bound to the membrane was visualized with a chemiluminescent kit purchased from BioRad Inc., according to the manufacturer's instructions (also see Blake M.S. et al.Anal. biochem. 136:175-179 (1984). Interaction of the of the antibody with the protein localized within cells in breast and ovarian tissue and other tissue obtained from patients was visualized by immunohistochemical methods.

### THE MOLECULES OF THE INVENTION

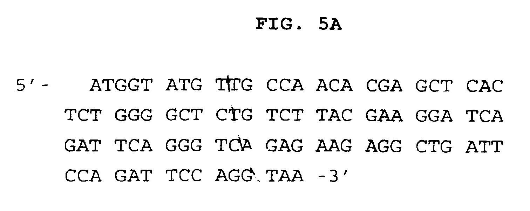

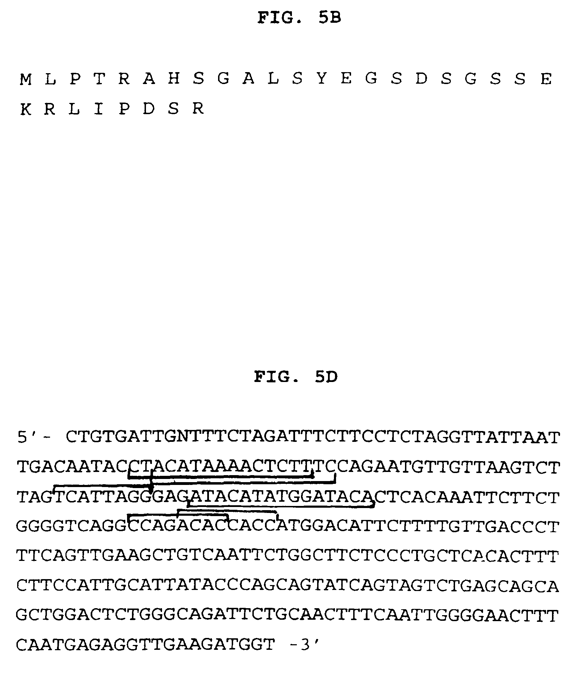

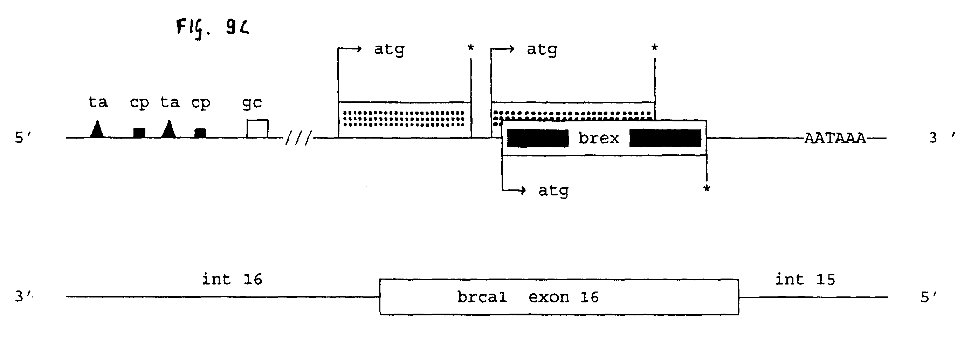

The invention relates to the discovery of a pb-gene (L-oncogene) the sequence and SEQ ID of which are given above, and the protein products of this gene (L-oncoprotein), the sequence and SEQ ID also given above, using the DGDPS procedure. The transcription of the L-oncogene is programmed by a promoter element located within the sequence shown in the embodiment of figure 5D (BREXL). The chromosomal location and selected potential biological features of features of the L-oncoprotein which may be relevant to a role in breast and ovarian cancer, are set forth in the examples provided below and in Table 1. The organization of three further genes designated BC531L, BC532L, BC533L deduced from our search procedure also herein described, is shown in figure 9a and 9b and the organisation of BREX gene is shown in figure 9c.

### Example 1

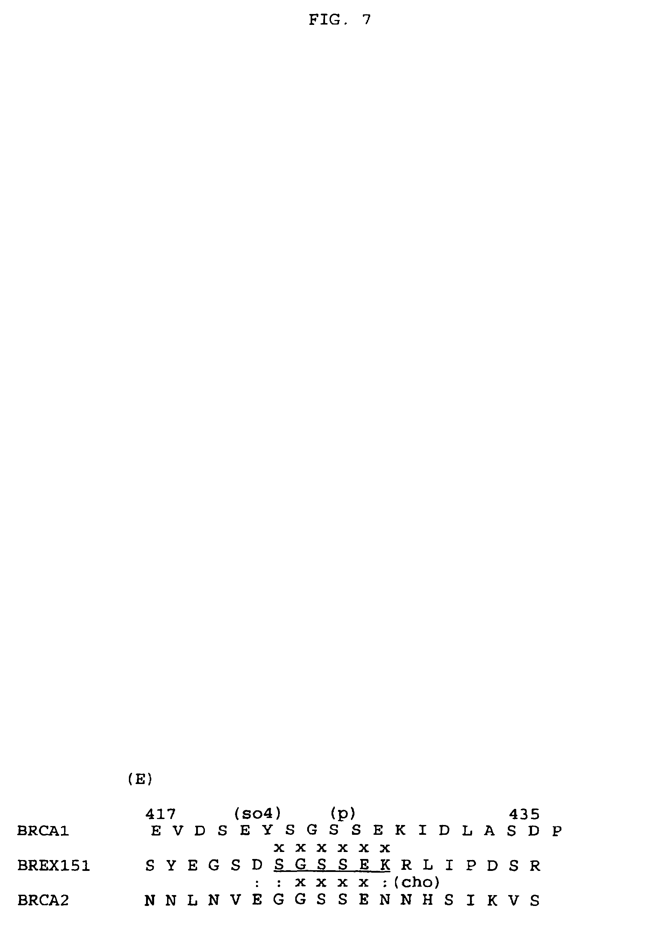

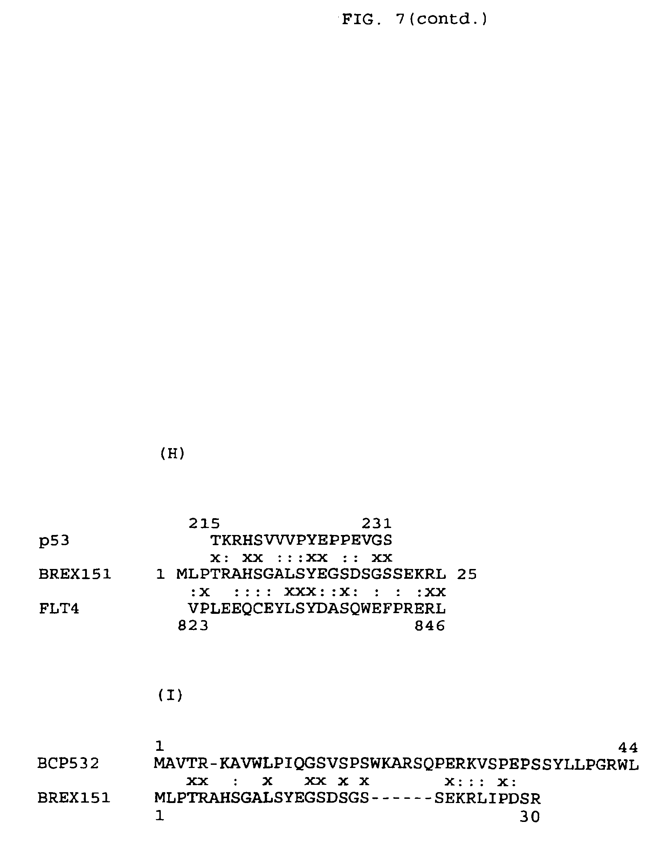

BREX (SEQ ID NO:29) is a tachykinin-like polypeptide hormone which can effect many intracellular systems including activation of expression of the other L-oncogenes described herein, and FLT4 tyrosine kinase. As shown in figure 7H, it has significant homology to domains in p53 and FLT4. It contains a domain which is identical to a similar domain in BRCA1 which is part of one of two BRCA1 sulphation sites: The same domain is also 100% homologous about (70 % similar) to a domain in BRCA2 which is adjacent to a major glycosylation site. Tyrosine sulphation is important for proteins that enter the golgi vesicles to be glycosylated. BRCA1/BRCA2 are glycosylated granin type proteins which are secreted; glycosylation occurs mainly in the golgi vesicles and is necessary for the functional integrity of these proteins. Hence BREX may be able to obstruct maturation and transport activity of BRCA1/BRCA2. This and other data driven deductions led to the conclusion that BREX was involved in the earliest stages of BC whereas the other L-oncoproteins were likely involved at later stages of the disease.

A sequence region containing a potential L-oncogene was selected using BC532 as an "evolutionary relationship" probe. The evolutionary relationship between the L-oncogenes and between the L-oncoproteins was done according to the method of Higgins D.G and Sharp P.M., CABIOS :5:151-153 (1989). The relatedness between L-oncogenes, and between L-oncoproteins are shown in figure 6a & 6b.

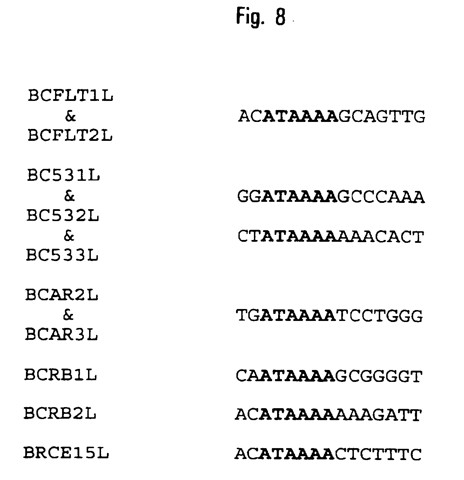

In another embodiment of the invention it was discovered that all L- oncogenes contained at least one promoter element in the 5' regulatory region which harboured a conserved 6 bp core promoter sequence as shown in the embodiment of figure 8. This indicated that at least one promoter for each L-oncogene might be influenced by the same transcription factor/ mechanism or same variety of factors. Blocking such a factor may be sufficient to stop transcription of all the L-oncogenes.

Furthermore, according to this invention, the combination of non expression in normal cells and the complexity of the promoter systems located in the 5' regulatory region of SEQ ID NO:31, is an indication that the promoter can be regulated by a number of factors probably in a tissue specific manner. Preventing activation of selective L-oncogene promoters is, therefore, an ideal method to prevent expression of transcription of L-oncogenes, and hence expression of L-oncoproteins.

### Example 2

Detection of BREX mRNA in breast cancer and normal patients.

mRNA was isolated and converted to cDNA as described. PCR was done with the appropriate primer pair as shown in figure 12 m the correct size fragment was amplified in all five BC patients but not in three normal people tested.

### Example 3

Detection of BREX protein and BREX protein complexed to human IgG in serum from an advanced breast cancer patient using the ELISA test described in figure 12 b/c . Serum from all breast and ovarian cancer positive patients was positive for BREX ; serum from four of five normal patients was negative for BREX and serum from 11 of 14 patients with other epithelial cell cancers and inflammatory diseases were negative for BREX serum one patient with pancreatic, one patient with stomach cancer and two benign BC patients were positive for BREX.

### Example 4

Detection of BRCA1 and BREX from tumour extract of the same patient.

Serum from a BC patient was analyzed by western blotting following non SDS cationic poly acrylamide gel electrophorosis and immunoreacting with anti-brex affinity purified epitope specific poly clonal IgG to detect BREX (figure 12 p and anti BRCA1 monoclonal antibody to detect BRCA1 using (figure 12 q). The immunoreactive bands were determined with a species specific chemiluminescent labelled monoclonal IgG.

Therefore, according to this invention it is possible to use PCR primers and mono specific antibodies directed against L-oncoprotein epitopes to detect, with very high level of confidence, breast cancer and ovarian cancer in humans. Since the L-oncoproteins have the combined and singular biological properties to cause many biochemical characteristics of epithelial cell derived cancers, eliminating these molecules can lead to cessation of the symptoms; therefore, L-oncoproteins are suitable targets against which therapeutic reagents to stop breast cancer in humans can be directed.

### Biochemical activity of the L-oncogenes relating to a role in breast and ovarian tumour formation: How L-oncogenes might initiate breast and ovarian cancer.

As can be seen from the expression profile of the L-oncogenes in tumour cells, shown in table 2a, BREXL was also present in all the specimens tested; anti-BREX antibody also detected BREX in all breast tumours tested (data not shown). In immunohistochemical experiments (data not shown here), done with anti BRCA1 antibodies provided by others, anti-BRCA1 and anti-BREXab1 antibody co-reacted with what appeared to be the same structures in the cytoplasm of breast tumour cells, whereas anti-BC531 appeared mainly in the nucleus and scattered throughout the cytoplasm. While anti-BRCA1 was located mainly in the nucleus in normal breast tissue, neither anti-BC531 nor anti-BREX reacted with cells from normal breast tissue. These results suggested to us that BRCA1 and BREX were closely associated in the cytoplasm of breast tumour cells (Chen, Y. et al, Science 270:789-791 (1995), have demonstrated conclusively that while BRCA1 is found in the nucleus in all normal cells, it is almost exclusively found in the cytoplasm in breast and ovarian cancer cells), whereas BC531 is associated with the nucleus in breast tumour cells. The latter supports the results shown in the embodiment of figure 12H and 12, which show that BC531 was detected in proteins isolated from a tumour cell nuclear pellet after treating the pellet with Triton x-100^{®}. Similar experiments have not been done with BRCA2, because a specific BRCA2 antibody was not available to us.

In summary the invention involves the discovery of a gene (BREX) expressed from within the region encoding the BRCA1 gene on chromosome 17. BREX encodes a protein which can be classified an endogenous pathogen as the human immune system produces a endogenous anti BREX antibody against it. The pathogenic activity of BREX stems from out competing BRCA1 and BRCA2 for sulphation and glycosylation sites respectively on the golgi membranes and the potential to mimic tachykinin type physiologically active peptides. The loss of BRCA1/BRCA2 leads to an activation cascade of a number of genes that are normally repressed in humans. The protein expressed by some of these genes may bind to sites on chromosomal DNA and influence the activation of critical developmentally and growth related genes and other pathogenic genes normally repressed in the human genome; at least one of these pathogenic genes might be involved in disrupting tyrosine kinase phosphorylation reactions.

The Uses of the Molecules of the Present Invention

### A. Diagnostic Uses

Since the L-oncogene BREX is not expressed at a detectable level by normal cells, the detection of this molecule in a tissue or fluid sample (such as a biopsy sample, or a blood or urine or saliva) is indicative of the presence of breast or ovarian cancer in a human.

In this invention we have used two types immunological approaches for detecting the BREX gum product in human material; however, the detection of these molecules may be done by any of a variety of immunological methods; a large number of suitable immunoassay formats have been described (Yolken, R.H., Rev. Infect. Dis. 4:35 (1982); Collins, W.P., In: Alternative Immunoassays, John Wiley & Sons, NY (1985); Ngo, T.T. et al., In: Enzyme Mediated Immunoassay, Plenum Press, NY (1985); incorporated by reference herein. Using epitope specific, double affinity purified, monospecific antibodies as shown in the examples given above and in figures 12A and 12B, the presence of the target molecule is detected by an immunopositive reaction; the intensity of which is proportional to the concentration of the antibody bound to the target molecule.

The other approach which is applicable to diagnosis of breast and ovarian cancer is detecting the expression of L-oncogene mRNAs BREXL in a breast cancer patient or in a randomly selected population as described in the examples given above. Diagnosis is based on any method capable of detecting mRNA encoding these proteins in tissue and biological fluids described herein. Thus, molecules nucleic acid probes (DNA or RNA) capable of hybridizing to such molecules may be used in the diagnosis of breast and ovarian cancer.

In one embodiment, the assays may be performed on RNA extracted from blood, cells or tissue as described in the specifications herein.

Where the concentration of such mRNA in a sample is too low to be detected, such mRNA may be specifically amplified through the use of any of a variety of amplification protocols, such as PCR (Mullis, K.B., Cold Spring Harbor Symp. Quant. Biol. 51:263-273 (1986); Mullis K. et al., U.S. Patent 4,683,202; Erlich, H., U.S. Patent 4,582,788; Saiki, R. et al., US 4,683,194 and Mullis, K.B., et al., Met. Enzymol. 155:335-350 (1987), or transcription-based amplification systems (Kwoh D et al., Proc. Natl. Acad. Sci. (U.S.A.) 86:1173 (1989); Gingeras TR et al., PCT appl. WO 88/10315; Davey, C. et al. European Patent Application Publication no. 329,822).

### B. Prognostic Uses

The present invention additionally provides a sensitive and specific of monitoring of possible recurrence of the disease in an individual believed to be cured of breast or ovarian cancer. Thus, any of the above-described assays may be performed on an asymptomatic individual in order to assess if there is a re-initiation of these diseases.

### C. Therapeutic Uses

Significantly, the present invention provides a means for treating Breast cancer. Such treatment may be either "prophylactic", one that is provided in advance of any clinical symptom BC in order to prevent or attenuate any subsequent onset of the disease or A therapeutic treatment one that is provided in response to the onset of a symptom of breast cancer, and serves to attenuate an actual symptom of the disease.

Treatment is provided by administering to a patient an effective amount of an antibody, or an antibody fragment (F(ab'), F(ab')2, or single chain antibodies, or pseudo-homologous antibodies including "humanized" antibodies or a combination of the above that is capable of neutralizing BREX. As used herein, an effective amount is an amount sufficient to mediate a clinically significant change in the severity of a symptom, or a clinically significant delay in the onset of a symptom.

These may include monospecific polyclonal or monoclonal antibodies (or fragments of either) may be administered. More preferably, such molecules will be of non-immunogenic. The latter can be pseudo-homologous (i.e. humanised molecules prepared by recombinant or other technology. Such antibodies are the equivalents of the monoclonal and polyclonal antibodies, but are less immunogenic, and are better tolerated by the patient.

Humanized anti BREX antibodies can be produced, for example by replacing an immunogenic portion of each antibody with a corresponding, but non-immunogenic portion (i.e. chimeric antibodies) . The production of humanized antibodies have been described in details in the following references (Robinson, R.R. et al., International Patent Publication PCT/US8 6/02269; Akira, K. et al., European Patent Application 184,187; Taniguchi, M., European Patent Application 171,496; Morrison, S.L. et al., European Patent Application 173,494; Neuberger, M.S. et al., PCT Application WO 86/01533; Cabilly, S. et al., European Patent Application 125,023; Better, M. et al., Science 240:1041-1043 (1988); Liu, A.Y. et al., Proc. Natl. Acad. Sci. USA 84:3439-3443 (1987); Liu, A.Y. et al., J. Immunol. 139:3521-3526 (1987); Sun, L.K. et al., Proc. Natl. Acad. Sci. USA 84:214-218 (1987); Nishimura, Y. et al., Canc.Res. 47:999-1005 (1987); Wood, C.R. et al., Nature 314:446-449 (1985); Shaw et al., J. Natl.Cancer Inst. 80:1553-1559 (1988); Morrison, S.L. (Science, 229:1202-1207 (1985); Oi, V.T. et al., BioTechniques 4:214 (1986); Jones, P.T. et al., Nature 321:552-525 (1986); Verhoeyan et al., Science 239:1534 (1988); Beidler, C.B. et al., J. Immunol. 141:4053-4060 (1988)).

In another embodiment the desired therapy may be obtained by targeting the nucleic acid molecules, specifically the promoter sequences BREXP4 of the present invention with "antisense" nucleic acid molecules (anti sense oligonucleotides). To act as an antisense oligonucleotide, the nucleic acid molecule must be capable of binding to or hybridizing with that portion of the molecule which mediates the translation of the target mRNA. Antisense oligonucleotides are disclosed in European Patent Application Publication Nos. 263,740; 335,451; and 329,882, and in PCT Publication No. WO90/00624.

As used herein, an "antisense oligonucleotide" is a nucleic acid (either DNA or RNA) whose sequence is complementary to the sequence BREXLP described herein, such that it is capable of binding to, or hybridizing with, an endogenous promoter or heat shock sequence in a manner sufficient to impair its transcription, and significantly inactivate it in a cell, and thereby impair (i.e. attenuate or prevent) its the translation into protein.

The anti sense oligonucleotides may be transported into the cell using the Protein-Polycation Conjugates system (Beug, H. et al United States patent 5,354, 844 11/10 1994) in an appropriate pharmaceutical compound.

Anti sense nucleic acid molecule(s) may be administered using viral or retroviral vectors in accordance with the methods of "gene therapy". The topic is extensively covered in: Biotechnology, A Comprehensive Treatise, volume 7B, Gene Technology, VCH Publishers, Inc. NY, pp 399-458 (1989)).

Although, as indicated above, such gene therapy can be provided to a recipient in order to treat (i.e. suppress, or attenuate) an existing condition, the principles of the present invention can be used to provide a prophylactic gene therapy to individuals who, are deemed at risk for the disease.

### Administration of the Molecules of the Present Invention

As disclosed in Remington's Pharmaceutical Sciences (1980), additional nucleic acid molecules may be employed to control the duration of action of any therapeutic substance administered to a patient.

Having now generally described the invention, through references and examples that makes it more readily understood by any one sufficiently skilled in the art, it must be pointed out that these are not intended to be limiting of the present invention, unless specified.

### Legend to figures 1-5

| | |
|---|---|
| over & under lined | = CAATT promoter element |
| underlined | = TATA promoter element |
| over lined | = Cap site |
| underline x 2 | = bipartite nuclear transport sequence |
| boxed | = transmembrane helix |
| x---Fe⁺⁺---x | = heme iron binding site |

### Legend to Figure 12

A. Sequence of epitopes used to raise monospecific polyclonal antibodies against L-oncoproteins.
B. ELISA test for detecting L-oncoproteins in human body fluids.
C. ELISA test for detecting endogenous anti L-oncoprotein antibody in human body fluids.
D. Detection of BC531 with BC531ab1 in metastatic breast tumours and normal breast, A1 - normal breast.
E. Detection of BC532 using BC532ab1, same as D.
F. Detection of BC531 with BC531ab1 in proteins isolated from 15 confirmed and 3 suspected primary breast tumours. B1 and B4 are from normal breast, A2, B2 and C2 were from suspected tumours.
G. Detection of BC532 using BC532ab1. A1 and B1 = normal breast, A2, B2 and C2 = suspected tumours.
H. Identification of tumour tissue from a mixture of normal and breast cancer tissue, using BC531ab1. A1, B1, C1, A2 and B2 were correctly identified as cancerous breast tissue; C2, D1 and D2 were normal.
I. Detection of BC532 using BC532ab1, same identification as H.
J. Detection of BC531 in nuclear and cytoplasmic fractions of breast tumours using BC531ab1. A = BC cytoplasmic fraction, B = BC nuclear fraction, C = normal nuclear fraction and D = normal cytoplasmic fraction.
K. Detection of BC532 using BCS32ab1, same as J.
O. Detection of BREX in tumour tissue from a metastatic BC patient: the protein extract was subjected to cationic non-SDS PAGE; BREX was detected by western blotting followed by reaction with anti-BREX1ab and a chemiluminescence mouse anti-rabbit IgG mab.
   Detection of BRCA1 in the same tumour extract as in "Q". BRCA1 protein was detected by anti-BRCA1 mouse maB an a chemiluminescence labelled anti rabbit IgG.

### SEQUENCE LISTING

(1) GENERAL INFORMATION:
   (i) APPLICANT: Bergmann, Johanna and Preddie, E. Rick
   (ii) TITLE OF INVENTION : Agents for pre-symptomatic detection, prevention and treatment of breast cancer.
   (iii) NUMBER OF SEQUENCES: 3
   (iv) CORRESPONDENCE ADDRESS:
      (A) ADDRESSEE: DR. J. BERGMANN
      (B) STREET: MÖRIKESTR. 22
      (C) CITY: HAMBURG
      (D) STATE:
      (E) COUNTRY: GERMANY
      (F) ZIP: 22587
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25
   (vi) CURRENT APPLICATION DATA:
      (A) APPLICATION NUMBER: PCT
      (B) FILING DATE: 30 November 1995
      (C) CLASSIFICATION:
   (viii) ATTORNEY/AGENT INFORMATION:
      (A) NAME: NONE
      (B) REGISTRATION NUMBER:
   (ix) TELECOMMUNICATION INFORMATION:
      (A) TELEPHONE: (4940) 862-576
      (B) TELEFAX: (4940) 862-596
(2) INFORMATION FOR SEQ ID NO:28:
   (i) SEQUENCE CHARACTERISTICS:
      (A) LENGTH: 95 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE: CLONE: BRCE151L
   (xi) SEQUENCE DESCRIPTION: SEQ ID NO:28:
   ATGGTATGTT GCCAACACGA GCTCACTCTG GGGCTCTGTC TTACGAAGGA 50
   TCAGATTCAG GGTCAGAGAA GAGGCTGATT CCAGATTCCA GGTAA 95
(2) INFORMATION FOR SEQ ID NO:29:
   (i) SEQUENCE CHARACTERISTICS:
      (A) LENGTH: 30 amino acids
      (B) TYPE: amino acid
      (D) TOPOLOGY: linear
   (ii) MOLECULE TYPE: proten
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151L
   (xi) SEQUENCE DESCRIPTION: SEQ ID NO:29:
(2) INFORMATION FOR SEQ ID NO:31:
   (i) SEQUENCE CHARACTERISTICS:
      (A) LENGTH: 235 base pairs
      (B) TYPE: nucleic acid
      (C) STRANDEDNESS: single
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151
   (xi) SEQUENCE DESCRIPTION: SEQ ID NO:31:
   TGACAATACC TACATAAAAC TCTTTCCAGA ATGTTGTTAA GTCTTAGTCA 50
   TTAGGGAGAT ACATATGGAT ACACTCACAA ATTCTTCTGG GGTCAGGCCA 100
   GACACCACCA TGGACATTCT TTTGTTGACC CTTTCAGTTG AAGCTGTCAA 150
   TTCTGGCTTC TCCCTGCTCA CACTTTCTTC CATTGCATTA TACCCAGCAG 200
   TATCAGTAGT CTGGAGCAGC AGCTGGACTC TGGGC                 235

### SEQUENCE LISTING

(1) GENERAL INFORMATION:
   (i) APPLICANT: Bergmann, Johanna and Preddie, E. Rick
   (ii) TITLE OF INVENTION: Agents for pre-symptomatic detection, prevention and treatment of breast cancer.
   (iii) NUMBER OF SEQUENCES: 8
   (iv) CORRESPONDENCE ADDRESS:
      (A) ADDRESSEE: DR. J. BERGMANN
      (B) STREET: MÖRIKESTR. 22
      (C) CITY: HAMBURG
      (D) STATE:
      (E) COUNTRY: GERMANY
      (F) ZIP: 22587
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: PCT
      (B) FILING DATE: 30 November 1995
      (C) CLASSIFICATION:
   (viii) ATTORNEY/AGENT INFORMATION:
      (A) NAME: NONE
      (B) REGISTRATION NUMBER:
   (ix) TELECOMMUNICATION INFORMATION:
      (A) TELEPHONE: (4940) 86662948
      (B) TELEFAX: (4940) 86662949
(2) INFORMATION FOR SEQ ID NO: 1
   (i) SEQUENCE CHARACTERISTICS:
      (A) LENGTH: 95 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (vii)IMMEDIATE SOURCE:
      CLONE: BRCE151L (BREXL)
   (xi) SEQUENCE DESCRIPTION: SEQ ID NO:1:
   ATGGTATGTT GCCAACACGA GCTGACTCTG GGGCTCTGTC 40
   TTCCGAAGGA TCAGATTCAG GGTCAGAGAA GAGGCTGATT 80
   CCAGATTCCA GGTAA 95
(2) INFORMATION FOR SEQ ID NO:2:
   (i) SEQUENCE CHARACTERISTICS:
      (A) LENGTH: 30 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151L(BREX)
   (xi) SEQUENCE DESCRIPTION: SEQ ID NO:2:
   Met Leu Pro Thr Arg Ala Asp Ser Gly Ala Leu Ser Ser Glu Gly
   1 5 10 15
   Ser Asp Ser Gly Ser Ser Glu Lys Arg Leu Ile Pro Asp Ser Arg
   20 25 30
(2) INFORMATION FOR SEQ ID NO:3:
   (i) SEQUENCE CHARACTERISTICS:
      (A) LENGTH: 26 amino acids
      (B) TYPE: amino acid
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE152
   (xi) SEQUENCE DESCRIPTION: SEQ ID NO:3:
   Met Val Cys Cys Pro Gln Glu Leu Thr Leu Gly Leu Cys Leu Gln
   1 5 10 15
   Lys Asp Gln Ile Gln Gly His Gln Arg Arg Gly
   20 25
(2) INFORMATION FOR SEQ ID NO:4:
   (i) SEQUENCE CHARACTERISTICS:
      (A) LENGTH: 34 amino acids
      (B) TYPE: amno acid
      (D) TOPOLOGY: linear
   (ii) MOLECULE TYPE: protein
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE153
   (xi) SEQUENCE DESCRIPTION: SEQ ID NO:4
   Met Asp Ile Leu Leu Leu Thr Leu Ser Val Glu Ala Val Asn Ser
   1 5 10 15
   Gly Phe Ser Leu Leu Thr Leu Ser Ser Ile Ala Leu Tyr Pro Ala
   20 25 30
   Val Ser Val Val
(2) INFORMATION FOR SEQ ID NO:5:
   (i) SEQUENCE CHARACTERISTICS:
      (A) LENGTH: 327 base pairs
      (B) TYPE: nucleic acid
      (C) STRANDEDNESS: double
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151
   (xi) SEQUENCE DESCRIPTION: SEQ ID NO:5
(2)INFORMATION FOR SEQ ID NO:6:
   (i) SEQUENCE CHARACTERISTICS:
      (A) LENGTH: 49 base pairs
      (B) TYPE: nucleic acid
      (C) STRANDEDNESS: double
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151
   (xi) SEQUENCE DESCRIPTION: SEQ ID NO: 6
(2 INFORMATION FOR SEQ ID NO:7:
   (i) SEQUENCE CHARACTERISTICS:
      (A) LENGTH: 53 base pairs
      (B) TYPE: nucleic acid
      (C) STRANDEDNESS: double
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151
   (xi) SEQUENCE DESCRIPTION: SEQ ID NO: 7
(1) INFORMATION FOR SEQ ID NO:8:
   (i) SEQUENCE CHARACTERISTICS:
      (A) LENGTH: 52 base pairs
      (B) TYPE: nucleic acid
      (C) STRANDEDNESS: double
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151
   vi) ORIGINAL SOURCE:
      (A) ORGANISM: HOMO SAPIENS
   (xi) SEQUENCE DESCRIPTION: SEQ ID NO: 8
   TTCTTTTGTT GACCCTTTCT GTTGAAGCTG TCAATTCTGG 40
   CTTCTCCCTG CT 52
(2)INFORMATION FOR SEQ ID NO:6:
   (i) SEQUENCE CHARACTERISTICS:
      (A) LENGTH: 49 base pairs
      (B) TYPE: nucleic acid
      (C) STRANDEDNESS: double
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151
   (xi) SEQUENCE DESCRIPTION: SEQ ID NO: 6
(2 INFORMATION FOR SEQ ID NO:7:
   (i) SEQUENCE CHARACTERISTICS:
      (A) LENGTH: 53 base pairs
      (B) TYPE: nucleic acid
      (C) STRANDEDNESS: double
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151
   (xi) SEQUENCE DESCRIPTION: SEQ ID NO: 7
(1) INFORMATION FOR SEQ ID NO:8:
   (i) SEQUENCE CHARACTERISTICS:
      (A) LENGTH: 52 base pairs
      (B) TYPE: nucleic acid
      (C) STRANDEDNESS: double
      (D) TOPOLOGY: linear
   (ii) MOLECULE TYPE: genomic DNA
   (iii) HYPOTHETICAL: NO
   (iv) ANTI-SENSE: NO
   (vi) ORIGINAL SOURCE:
      (A) ORGANISM: HOMO SAPIENS
   (vii) IMMEDIATE SOURCE:
      (B) CLONE: BRCE151
   vi) ORIGINAL SOURCE:
      (A) ORGANISM: HOMO SAPIENS
   (xi) SEQUENCE DESCRIPTION: SEQ ID NO: 8
   TTCTTTTGTT GACCCTTTCT GTTGAAGCTG TCAATTCTGG 40
   CTTCTCCCTG CT 52

## Claims

1. A nucleic acid molecule free of natural contaminants selected from the group consisting of brex and brex promoter the sequence of which is SEQ ID NO:28 and SEQ ID NO:31, respectively.

2. A protein molecule free of natural contaminants consisting of BREX the sequence of which is SEQ ID NO:29.

3. An antibody specific for BREX the sequence of BREX being SEQ ID NO:29.

4. The antibody of claim 3, wherein the antibody is produced by immunizing appropriate animals with polypeptide domains of BREX.

5. The antibody of claim 3 specific for BREX wherein the antibody is produced endogenously by a human.

6. An antibody or an antibody fragment, or single chain antibodies, or a combination thereof, capable of binding specifically to BREX for use in the prophylactic or therapeutic treatment of breast cancer.

7. Use of an antibody, or an antibody fragment or of a single chain antibody or a combination thereof, capable of binding specifically to BREX for the manufacture of a medicament for the prophylactic treatment of breast cancer.

8. An antibody as defined in claim 6 wherein the antibody is a monospecific polyclonal antibody or a monoclonal antibody or a recombinant antibody or a humanized antibody.

9. An antisense RNA specific for the molecule having the sequence complementary to SEQ ID NO: 28, i.e. for brex mRNA or brex promoter the sequence of this letter molecule being SEQ ID NO:31.

10. Use of an antisense RNA of claim 9 capable of binding specifically to brex mRNA or brex promoter for manufacture of a medicament for the prophylactic treatment of breast cancer.

11. A method for the diagnosis or the presymptomatic diagnosis of breast cancer comprising detecting in a sample of tissue or a fluid a protein as defined by SEQ ID NO:29 or comprising detecting of brex mRNA as defined by its cDNA having SEQ ID NO:28.

12. A method for the diagnosis or the presymptomatic diagnosis of breast cancer comprising detecting in a fluid sample an antibody specific for the BREX protein as defined by SEQ ID NO:29.

13. An antibody as defined in claims 6 or 8, wherein the disease is breast cancer or epithelial cell cancers originating from breast cancer.

14. The use as defined in claim 7 wherein the antibody is as defined in claim 8.

15. The use as defined in claims 11 or 12 wherein the disease is as defined in claim 13.

## Patentansprüche

1. Ein Nukleinsäuremolekül frei von natürlichen Kontaminationen und ausgewählt aus einer Gruppe, die aus brex und brex Promotor besteht, deren Sequenzen SEQ ID NR:28 bzw. SEQ ID NR:31 sind.

2. Ein Proteinmolekül frei von natürlichen Kontaminationen, bestehend aus BREX, dessen Sequenz SEQ ID NR:29 ist.

3. Ein Antikörper mit Spezifität für BREX, wobei die Sequenz von BREX SEQ ID NR:29 ist.

4. Der Antikörper von Anspruch 3, wobei der Antikörper durch limmunisieren eines geeigneten Tiers mit Polypeptiddomänen von BREX hergestellt wird.

5. Der für BREX spezifische Antikörper von Anspruch 3, wobei der Antikörper endogen in einem Menschen produziert wird.

6. Ein Antikörper oder ein Antikörper-Fragment, oder Einzelketten-Antikörper, oder eine Kombination aus diesen, welche spezifisch an BREX binden können, zur Verwendung in der prophylaktischen oder therapeutischen Behandlung von Brustkrebs.

7. Der Gebrauch eines Antikörpers oder eines Antikörper-Fragments, oder eines Einzelketten-Antikörpers, oder einer Kombination aus diesen, welche spezifisch an BREX binden können, zur Herstellung eines Medikaments für die prophylaktische Behandlung von Brustkrebs.

8. Ein Antikörper wie in Anspruch 6 definiert, wobei der Antikörper ein monospezifischer polyklonaler Antikörper oder ein monoklonaler Antikörper oder ein rekombinanter Antkörper oder ein humanisierter Antikörper ist.

9. Eine Gegensinn- (antisense) RNS mit Spezifität für das Molekül mit der komplementären Sequenz zu SEQ ID NR:28, d.h. für brex mRNS oder brex Promotor, dessen Sequenz die SEQ ID NR:31 ist.

10. Die Verwendung einer Gegensinn-RNS von Anspruch 9, welche spezifisch an brex mRNS oder brex Promotor binden kann, für die Herstellung eines Medikamentes zur prophylaktischen Behandlung von Brustkrebs.

11. Eine Methode zur Diagnose oder präsymptomatischen Diagnose von Brustkrebs, bestehend aus dem Nachweis eines durch SEQ ID NR:29 definierten Proteins in einer Gewebe- oder Flüssigkeitsprobe, oder bestehend aus dem Nachweis von brex mRNS wie definiert durch seine cDNS mit der Sequenz SEQ ID NR:28.

12. Eine Methode zur Diagnose oder präsymptomatischen Diagnose von Brustkrebs, bestehend aus dem Nachweis eines Antikörpers in einer Flüssigkeitsprobe, welcher Spezifität für das BREX Protein wie definiert durch SEQ ID NR:29 hat.

13. Ein Antikörper wie definiert in den Ansprüchen 6 oder 8, wobei die Krankheit Brustkrebs oder von Brustkrebs herrührender Epithelzellkrebs ist.

14. Die Verwendung wie definiert in Anspruch 7, wobei der Antikörper ist wie in Anspruch 8 definiert.

15. Die Verwendung wie definiert in den Ansprüchen 11 oder 12, wobei die Krankheit ist wie in Anspruch 13 definiert.

## Revendications

1. Une molécule d'acide nucléique sans contaminant naturel faisant partie du groupe constitué de brex et du promoteur de brex dont les séquences sont respectivement SEQ ID NO : 28 et SEQ ID NO : 31.

2. Une protéine sans contaminant naturel constituée de BREX dont la séquence est SEQ ID NO:29.

3. Un anticorps spécifique de BREX dont la séquence est SEQ ID : 29.

4. L'anticorps revendiqué en 3 lorsqu'il est produit par immunisation d'animaux convenables au moyen de domaines polypeptidiques de BREX.

5. L'anticorps revendiqué en 3 spécifique de BREX lorsqu'il est produit de façon endogène chez l'humain.

6. Un anticorps, un fragment d'anticorps, des anticorps simple chaîne ou une combinaison de ceux-ci capables de se lier spécifiquement à BREX afin de traiter le cancer du sein de façon prophylactique ou thérapeutique.

7. Un anticorps, un fragment d'anticorps, des anticorps simple chaîne ou une combinaison de ceux-ci capables de se lier spécifiquement à BREX afin de fabriquer un médicament pour traiter le cancer du sein de façon prophylactique ou thérapeutique.

8. Un anticorps tel que défini en 6 lorsqu'il s'agit d'un anticorps polyclonal monospécifique, d'un anticorps monoclonal, d'un anticorps recombinant ou d'un anticorps humanisé.

9. Un ARN antisens spécifique pour la molécule de séquence complémentaire de SEQ ID NO : 28, c'est-à-dire pour l'ARNm de brex ou le promoteur de brex, dont la séquence est AES ID NO:31.

10. L'utilisation de l'ARN antisens revendiqué en 9 capable de se lier spécifiquement à l'ARNm de brex ou à son promoteur afin de fabriquer un médicament pour traiter le cancer du sein de façon prophylactique.

11. Une méthode de diagnostic ou de diagnostic présymptomatique du cancer du sein comprenant la détection dans un échantillon de tissu ou un liquide biologique, une protéine définie par la SEQ ID NO : 29, ou bien la détection de l'ARNm de brex tel que défini par son ADNc dont la séquence est SEQ ID NO : 28.

12. Une méthode de diagnostic ou de diagnostic présymptomatique du cancer du sein comprenant la détection dans un échantillon de liquide biologique d'un anticorps spécifique de la protéine BREX définie par SEQ ID NO : 29.

13. Un anticorps tel que revendiqué en 6 ou 8 lorsque la maladie est le cancer du sein ou des tumeurs de cellules épithéliales provenant du cancer du sein.

14. L'utilisation revendiquée en 7 lorsque l'anticorps est celui revendiqué en 8.

15. L'utilisation revendiquée en 11 lorsque la maladie est celle revendiquée en 13.
